Europäisches Patentamt

European Patent Office

Office européen des brevets

(19)

(11) Publication number : **0 455 422 A2**

# EUROPEAN PATENT APPLICATION

(12)

(21) Application number : 91303756.0

(51) Int. Cl.⁵ : **A61K 7/06**

(22) Date of filing : 25.04.91

(30) Priority : 27.04.90 US 515381

(43) Date of publication of application :
06.11.91 Bulletin 91/45

(84) Designated Contracting States :
CH DE FR GB IT LI NL

(71) Applicant : MERCK & CO. INC.
126, East Lincoln Avenue P.O. Box 2000
Rahway New Jersey 07065-0900 (US)

(72) Inventor : Mellin, Theodore M.
32 Overhill Drive
North Brunswick, NJ 08902 (US)
Inventor : Thomas, Kenneth A., Jr.
245 Washington Avenue
Chatham Burough, NJ 07928 (US)

(74) Representative : Thompson, John Dr. et al
Merck & Co., Inc. European Patent
Department Terlings Park Eastwick Road
Harlow, Essex CM20 2QR (GB)

(54) Method of treating or preventing baldness with compositions containing fibroblast growth factor.

(57) A method of retarding or reversing baldness which comprises the topical application of compositions containing as at least one of its active in-ingredients fibroblast growth factor (FGF). The FGF may be acidic FGF (aFGF), basic FGF (bFGF), int-2, hst/KS3, FGF-5, FGF-6 or KGF or a combination thereof. The compositions may also include other protein growth factors or hair growth promoting agents combined with FGF.

EP 0 455 422 A2

## BRIEF DESCRIPTION OF TH DRAWING

The figure illustrates the uptake of 3H-thymidine into hair follicle DNA following treatment with aFGF.

## BACKGROUND OF THE INVENTION

Hair is produced by matrix cells within the hair follicle during a cycle of alternating periods of rest (telogen phase) and activity (anagen phase). It is during the relatively long anagen phase, three to six years in a teenager, that follicular cells divide and produce hair. Once hair is produced the cycle progresses to the relatively short telogen phase, about three months, during which cell division stops, hair growth ceases and the attachment of the hair to the follicle become weaker until the old hair is shed, Orentreich and Durr, Clin. in Plast. Surg. 9: 197-205 (1983). The cycle will repeat itself unless there is an interruption resulting from specific genetic factors, aging or disease.

Interruption of the hair cycle may result in baldness or alopecia. Balding is generally characterized by an increase in cycles with the anagen phase shortened with each cycle resulting in a rapid diminution in amount of hair and length of hair. The most common type of hair loss in humans, including both males and females, is termed androgenetic alopecia or male pattern baldness. Alopecia results from the gradual miniaturization of the long, coarse and pigmented terminal hairs to the very fine, non-pigmented and short vellus hairs due to the dimunition in the anagen phase with repeated cycles. This transition in hair type is associated with and results from an alteration of the hair cycle, Ebling and Hale, In, Biochem. & Physiol. Of The Skin, Ed. Goldsmith, Oxford Univ. Press (1983), pgs 522-552. Baldness results in a diminution of the size of hair follicles without a decrease in follicular numbers. Small telogen follicles found in balding skin are not devoid of hair but contain the very fine vellus hairs. Consequently, baldness is not considered a degenerative phenomenon, but a series of progressive changes in the scalp which results in the conversion of terminal hairs to velus hairs.

Currently there is no proven pharmaceutical treatment for this diminution in the size of hair follicles which results in baldness. The only pharmaceutical agent which has shown promise as a treatment for baldness is the vasodilator minoxidil. Minoxidil and related compounds have been shown to stimulate the conversion of vellus hair growth to terminal hair growth and to stimulate the growth rate of terminal hair, U.S. Patent 4,139,619. Even though minoxidil has been shown to stimulate hair growth or regrowth there is still a general concern that systemic side-effects can result, Franz, Arch, Dermatol. 121:203-206 (1985), Novak et al., Int. J. Dermatol 24:82-87 (1985). A minoxidil metabolite, minoxidil glucuronide, has been found suitable for topical application. Even though this compound has the potential for fewer side effects it still suffers from the undesired characteristic of stimulating hair growth in only about one third of the patients treated.

Vasoactive peptides have also been shown to stimulate hair growth. Substance P, a peptide containing 11 amino acids, and a Substance P-related peptide, when applied topically, were able to stimulate the regrowth of animal hair and to increase the temperature of the "underlying true skin layer in mice", European Patent Application 221,208. The digestive tract peptide hormone vasoactive intestinal polypeptide has been shown to have similar pharmacological activities, notably the regrowth of animal hair and the increase in skin temperature, European patent Application 225,639. Hockel et al., Int. J. Tiss. Reac. IV:323-331 (1984), have demonstrated that an angiogenic peptide termed monocyangiotropin (MAT) could induce hair growth when applied to rabbit skin. The peptide has a molecular weight of 4,500 dalton and induces the morphogenesis of new blood vessel patterns at the site of application. Histologically, numerous capillary-like vessels developed from existing vessels and the quiescent telogen hair follicles became activated to growing anagen follicles within a period of 1-2 weeks.

Animal organ preparations containing crude or partially purified growth factors have been used to study hair growth in rodents and growth kinetics in human hair bulb papilla cells and root sheath fibroblasts cultured in vitro. Mouse submaxillary gland epidermal growth factor (EGF) retarded the hair growth in neonatal mice that received EGF from birth through 14 days, Moore et al., J. Endocr. 88: 293-299 (1981). Mouse submaxillary gland EGF and bovine pituitary gland FGF were able to stimulate in vitro cell growth of human papilla cells and human root sheath fibroblasts, Katsuoka et al., Arch Dermatol. Res. 279: 247-250 (1987). Minoxidil was ineffective on both cell groups.

## OBJECTS OF THE INVENTION

It is, accordingly, an object of the present invention to provide a method in which fibroblast growth factor (FGF) is used for the treatment of existing baldness or to prevent the balding process. A further object is to use either acidic FGF (aFGF) or basic FGF (bFGF) or a combination containing both for the treatment of baldness. A further object is to provide a method for treating and preventing baldness in which FGF, either aFGF

EP 0 455 422 A2

or bFGF or a combination containing both, is combined with other growth factors or known hair growth stimulating compounds for the treatment of baldness. A further object is to provide compositions and methods for treating and preventing baldness which, while effective for said treatment are essentially non-toxic and relatively free of unwanted side effects. These and other objects of the present invention will be apparent from the following description.

## SUMMARY OF THE INVENTION

A method of retarding or reversing baldness which comprises the topical application of compositions containing as at least one of its active in-ingredients fibroblast growth factor (FGF). The FGF may be acidic FGF (aFGF), basic FGF (bFGF), int-2, hst/KS3, FGF-5, FGF-6 or KGF or a combination thereof. The compositions may also include other protein growth factors or hair growth promoting agents combined with FGF.

## DETAILED DESCRIPTION OF THE INVENTION

The present invention relates to the use of purified native, recombinant and mutated microheterogenous forms of human fibroblast growth factors (FGFs) and fragments thereof which retain the biological activity of native FGF for the treatment or prevention of baldness. The definition of FGF used herein further includes all allelic forms of FGF and fragments thereof which show equal or enhanced biological activity when compared to native FGF. A variety of terms have been used to describe growth factors which have been identified as being very similar, if not identical, to either aFGF or bFGF. Growth factors identical to acidic FGF include, but are not limited to, endothelial cell growth factor (ECGF); eye-derived growth factor II (EDGFII); heparin-binding growth factor $\alpha$ or 1 (HGF$\alpha$ or 1, HBGF$\alpha$ or 1); brain-derived growth factor (BDGF); retinal-derived growth factor (RDGF) and astroglial growth factor 1 (AGF1). Growth factors identical to basic FGF include, but are not limited to, eye-derived growth factor I (EDGF I); heparin-binding growth factor $\beta$ or 2 (HGF $\beta$ or 2, HBGF $\beta$ or 2); chondrosarcoma-derived growth factor; hepatoma-derived growth factor and astroglial growth factor 2 (AGF2).

Fibroblast growth factor is actually a family of proteins which share considerable amino acid sequence homology generally ranging from about 35 to about 55% identity in commonly aligned regions and have two similarly positioned introns in their genes, Thomas, Trends in Biochemical Sciences, 13:327-328 (1988). All family members stimulate mitogenesis of a variety of cells of mesodermal and ectodermal origin and generally bind to heparin, Thomas and Gimenez-Gallego, Trends in Biochem. Sci. 11: 81-84 (1986), Thomas, Federation J. 1: 434-440 (1987) and Thomas, Trends in Biochem. Sci., Supra. The present invention discloses that large molecular weight growth factors which exhibit multiple physiological activities, such as fibroblast growth factors, are able to retard or reverse alopecia or baldness by mitogenic and angiogenic activity, Thomas, Trends in Biochem. Sci. 13:327-328 (1988).

The FGF family is well known in the art and consists of the prototypic aFGF and bFGF, along with the recently described int-2, hst/KS3, FGF-5, FGF-6 and KGF see Thomas, Trends in Biochem. Sci., supra; Maries et al., Oncogene 4: 335-340 (1989) and Finch et al., Science 245: 752-755 (1989). The major human species of haFGF and hbFGF are isolated and purified from human cells or tissue by processes known in the art. Human aFGF is purified by the method of Thomas, U.S. Patent 4,444,760 while hbFGF is purified by the technique of Lemmon and Bradshaw, J. Cell Biochem. 21: 195-208 (1983) or Bohlen et al., Proc. Natl. Acad. Sci. USA 81: 5364-5368 (1984). The amino acid sequences for haFGF and hbFGF have been described by Gimenez-Gallego et al., Biochem, Biophys. Res. Comm. 130: 611-617 (1986) and Abraham et al., EMBO J. 5: 2523-2528 (1986) respectively. The minor FGF species, int-2, hst/KS3, FGF-5, FGF-6 and KGF are produced from isolated genes and the amino acid sequences deduced from the nucleotide sequences, Yoshida et al., Proc. Natl. Acad. Sci. USA 84: 7305-7309 (1987), Delli Bovi et al., Cell 50: 729-737 (1987), Zhan et al., Mol. Cell Biol., 8:3487-, Maries et al., Oncogene 4: 335-340 (1989) and Finch et al., Science 245: 752-755 (1989). Fibroblast growth factor (FGF) as used herein is contemplated to include one or more of the aforementioned FGFs. The preferred FGFs of the present invention are haFGF and hbFGF.

The present invention is contemplated to further include recombinant FGFs (r-FGFs) which are known in the art and have been produced by procedures which are well known in the art, such as: r-haFGF, Linemeyer et al., European Patent Application, Publication No. 259,953; r-hbFGF, Fiddes and Abraham, PCT, International Publication No. W087/01728. Recombinant forms of the minor species are produced by similar means using either the described genes or a nucleotide sequence obtained from the amino acid sequence combined with the Linemeyer et al., Fiddes and Abraham procedures or other procedures known in the art. It is also well known in the art relating to recombinant expression of polypeptides in bacteria that the expressed polypeptide will frequently contain an amino terminal methionine. The recombinant FGFs of the present invention when expressed in a bacterial system such as Escherichia. coli may generally contain a methionine as the first amino acid resi-

4

due. Polypeptides expressed in other cell systems may not have a methionine at the first position.

The present invention is further contemplated to include all microheterogeneous forms of the various human FGFs. Human acidic fibroblast growth factor typically exists in three microheterogeneous forms; a 154 amino acid form, a 140 amino acid form and a 139 amino acid form, Linemeyer, et al., European Patent Application, Publication No. 259,953. Human bFGF also appears to exist in three microheterogeneous forms; a 154 amino acid form, a 146 amino acid form and a 131 amino acid form, Fiddes and Abraham, PCT, International Publication No. W087/01728. Microheterogeneous forms as used herein refer to a single gene product, that is a peptide produced from a single gene unit of DNA, which is structurally modified at the mRNA level or following translation. These structural modifications, however, do not result in any significant alterations of biological activity of the peptide. Biological activity and biologically active are used interchangeably and are herein defined as the ability of native or recombinant hFGF to retard or reverse baldness. This may be accomplished by stimulating matrix cells or other cells associated with the hair follicle so that the anagen phase of hair growth is allowed to continue or to shorten the telogen phase. Biological activity may also be defined as a continuation of the telogen phase and anagen phase cycle so that normal hair growth is continuous. The microheterogeneous modifications may take place either in vivo or during the isolation and purification process. In vivo modification results from, but is not limited to, proteolysis, glycosylation, phosphorylation or acetylation at the N-terminus. Proteolysis may include exoproteolysis wherein one or more terminal amino acids are sequentially, enzymatically cleaved to produce microheterogeneous forms which have fewer amino acids than the original gene product. Proteolysis may also include endoproteolytic modification that results from the action of endoproteases which cleave the peptide at specific locations within the amino acid sequence. Similar modifications can occur during the purification process which also result in production of microheterogeneous forms. The most common modification occurring during purification is proteolysis which is generally held to a minimum by the use of protease inhibitors.

The present invention is also contemplated to include all biologically active mutated forms of FGF which contain a substitution or deletion of amino acids capable of forming extraneous intramolecular or intermolecular covalent bonds and oxidation susceptible amino acids. Substitution as used herein refers to a deliberate change in the DNA base sequence of aFGF such that a desired amino acid is substituted for an undesired amino acid. The undesired amino acid may be one which forms unwanted covalent or non-covalent bonds, especially disulfide bonds, or one which is oxidizable either of which may decrease the biological activity of the molecule. A deletion as used herein refers to a deliberate change in the DNA base sequene of FGF resulting in the elimination of the unwanted amino acid. The primary amino acid associated with intramolecular and intermolecular covalent bond formation is cysteine while the amino acids which are oxidization prone include cysteine, methionine and tryptophan. The cysteine residue or residues may be replaced with any amino acid which will not form disulfide bonds. The preferred amino acid for the substitution of cysteine is serine. The oxidation prone amino acids are replaced with any amino acid which is oxidation resistant, this includes, but is not restricted to, alanine, valine, leucine and isoleucine. Mutated FGF forms are produced by techniques well known in the art. An example of such a technique, site directed mutagenesis, is illustrated in European Patent Application, Publication No. 259,953.

Hair growth is stimulated at or near chronic cutaneous ulcers that have been treated with aFGF and exhibit healing. Chronic cutaneous ulcers are induced on the depilated dorsa of anesthetized Hartley strain guinea pigs by inserting a rubber tip of a plunger from a 10ml disposable syringe (insert) through an a 5 cm trans-scapular incision. The rubber tip and the skin above it are tightly encircled with an 0 ring preventing blood flow to a 5 cm square circle of skin over the insert. The incision is closed with wound staples. Acidic FGF treatment is initiated about 24 hr after removal of the 0 ring and insert (day 1). Duoderm dressings, impregnated with about 1.0 μg aFGF and heparin were changed daily for 7 days and on alternate days thereafter. At day 16, hair regrowth was much greater on the periphery of aFGF treated dermal ulcers than on the periphery of vehicle treated ulcers.

Fibroblast growth factor stimulates mitotic activity in human hair follicles maintained in vitro. Freshly plucked anagen stage human hair follicles are removed and washed with an acceptable washing solution, buffer or cell culture media. Acceptable washing solutions include, but are not limited to distilled water, physiological saline; while acceptable buffers include, but are not limited to, phosphate buffer, phosphate buffered saline (PBS), phosphate buffered saline glucose, TRIS-HCl and the like. Cell culture media include, but are not limited to, Eagle's minimal essential medium, Eagle's basal medium and Dulbecco's modified Eagle medium and the like, with Dulbecco's modified Eagle medium being preferred. The washed follicles are placed in cell culture plates, about 1 to about 10 follicles per well. The follicles are cultured in a culture medium generally supplemented with heat-inactivated serum, preferably about 10% fetal calf serum, antibiotics, preferably penicillin G, about 10,000 μl and about 10,000 μg streptomycin sulfate and about 1% fungizone. The cell culture of choice is Dulbecco's modified Eagle medium. The hair follicles are cultured for about 1 to about 5 days at 37°C in an atmos-

phere of about 5% $CO_2$ and about 95% air. All FGF concentrations are carried out in triplicate with experimental groups receiving about 0.01 to about 1.0 µg FGF/ml per day. When aFGF is assayed, heparin is added to each well at a ratio of about 3:1 heparin to FGF. Human serum albumin (HSA) is added to all wells at a ratio of about 10:1 HSA to FGF. Cell stimulation was determined by the addition of ³H-thymidine, about 5 µCi, about 0.14 µg/well, followed by the addition of cold thymidine, about 14.2 µg/well for the final 24 hr of incubation. Hair follicle DNA is precipitated with about 5% trichloroacetic acid and solubilized overnight at about 45°C in about IN NaOH. Scintillation counts are conducted in triplicate on 50 µl volumes of the final digests. Fibroblast growth factor stimulates DNA synthesis in anagen hair follicles. A dose response relationship is evident for follicles incubated 1 day.

Heparin is a sulfated glycosaminoglycan containing the sugars D-glucosamine and D-glucuronic acid which are sulfated to varying degrees. It is commercially available in a dry form as well as in a solution form for direct therapeutic utilization. When heparin is administered with aFGF in topical applications, the preferred concentration is from about 0.1 times to about 100 times the amount (mass) of aFGF administered per day.

For the treatment of baldness resulting from alopecia, FGF is administered in the form of a pharmaceutical composition comprising the FGF in combination with a pharmacologically acceptable carrier adapted for topical administration. The pharmaceutical composition may include aFGF, bFGF, int-2, hst/KS3, FGF-5, FGF-6 or KGF or a combination thereof. Fibroblast growth factor may be administered either with or without heparin, preferably with heparin when aFGF is included in the pharmaceutical composition. Topical administration of FGF, either alone or in combination will be in a solution, cream, ointment, gel, lotion, shampoo or aerosol formulation adapted for application to the skin. These topical pharmaceutical compositions containing FGF ordinarily include about 0.01 µg to about 100 µg/day/cm², preferably about 0.1 µg to about 10 µg/day/cm² of the active compound in a pharmaceutically acceptable carrier. Combinations of FGF may include any ratio of two or more compounds that will effectively stimulate hair growth or retard hair loss, such as for two FGFs 50:50, 25:75, 75:25 and the like. The total concentration of FGF in the combinations generally will not exceed about 0.01 µg to about 100 µg/day/cm², preferably about 0.1 µg to about 10 µg/day/cm² of the active compound in a pharmaceutically acceptable carrier.

The hair growth promoting activity of FGF may be enhanced or augmented by co-administration with other purified or recombinant derived protein growth factors (pGFs) which are well known in the art. Growth factors which may enhance or augment the stimulatory effect of FGF include, but are not limited to, members of the epidermal growth factor (EGF), platelet-derived growth factor (PDGF), transforming growth factor-alpha (TGF-α), transforming growth factor-beta (TGF-β), insulin-like growth factor (IGF), and vascular endothelial cell growth factor families, Van Brunt and Klausner, Biotechnology 6:25-30 (1988). The following references describe EGF, hEGF, and/or processes for obtaining them in pure form from natural sources or producing them by recombinant technology: Camble et al. U.S. Patent No. 3,914,824; Cohen et al., U.S. Patent No. 3,948,875; Nishimura et al., U.S. Patent No. 4,528,186; Bell, Published PCT patent application WO/85/00369; Urdea et al., Proc Natl. Sci. USA 80: 7461-7465 (1983). The following references describe PDGF, hPDGF, and/or processes for obtaining them in pure form from natural sources or producing them by recombinant technology: Lipton and Kepner, U.S. Patent No. 4,350,687; Betsholtz et al., Nature ,320,: 695-699 (1986); Johnsson et al., EMBO J. 3: 921-928 (1984); Stroobant and Waterfield, DMBO J. 3: 2963-2967 (1984). The following references describe TGF-α, hTGF-α, and/or processes for obtaining them in pure form from natural sources or producing them by recombinant technology: Derynck and Goeddel, European patent Application, publication No. 154,434; Derynck et al., Cell 38: 287-297 (1984). The following references describe TGF-β, hTGF-β and/or processes for obtaining them in pure form from natural sources or producing them by recombinant technology: Seyedin et al., U.S. Patent No. 4,774,228; Seyedin et al., U.S. Patent No. 4,774,322; Madison et al., DNA 7: 1-8 (1988); Marquardt et al., J. Biol. Chem. 262: 12127-12131 (1987). The following references describe IGF-I, hIGF-I and/or processes for obtaining them in pure form from natural sources or producing them by recombinant technology: Antoniades et al., Published PCT patent application WO/88/03409; Ikuo et al., European Patent Application, Publication No. 264,074 and Ueda et al., European Patent Application, Publication No. 219,814. The following references describe IGF-II, hIGF-II and/or processes for obtaining them in pure form from natural sources or producing them by recombinant technology: Shaar and Smith, European Patent Application, Publication No. 280,460. The co-administration of protein growth factors may have advantages over the administration of FGF alone or other PGFs alone. Co-administration of FGF with other protein growth factors will enhance the stimulation of various cell types which will result in increased follicle cell growth which can induce the conversion of telogen follicles to anagen follicles. This will lead to an increase in the number of terminal hairs and result in the reversal of the balding process.

The present invention relates to the use of FGF co-administered with other protein growth factors for the treatment of baldness resulting from alopecia. Fibroblast growth factor is co-administered in the form of a pharmaceutical composition comprising FGF and one or more PGFs in combination with a pharmacologically acceptable carrier adapted for topical administration. The pharmaceutical composition may include either aFGF or bFGF or a combination of the two. Fibroblast growth factor may be administered either with or without heparin, preferably with heparin when aFGF is included in the pharmaceutical composition. Topical administration of FGF, either aFGF or bFGF alone or in combination co-administered with PGFs will be in a solution, cream, ointment, gel, lotion, shampoo or aerosol formulation adapted for application to the skin. These topical pharmaceutical compositions containing pGFs will ordinarily include about 0.01 rg to about 100 μg/day/$^2$cm , preferably about 0.1 rg to about 10 μg/day/cm$^2$ of the active compound in a pharmaceutically acceptable carrier. Combinations of FGF and PGFs may include any ratio of two or more of the compounds that will effectively stimulate hair growth or retard hair loss. The total concentration of PGF to be co-administered generally will not exceed about 0.02 μg to about 200 μg/day/cm$^2$, preferably about 0.2 μg to about 20 μ/day/cm$^2$ of the active compounds.

The hair growth promoting activity of FGF may also be enhanced or augmented by co-administration with compounds or agents demonstrating hair growth promoting activity. These hair growth promoting agents include, but are not limited to, minoxidil, minoxidil derivatives or analogs and related compounds or analogs along with anti-androgens and 5α-reductase inhibitors. Fibroblast growth factor is co-administered in the form of a pharmaceutical composition comprising FGF and one or more of the compounds or agents demonstrating hair growth promoting activity in combination with a pharmacologically acceptable carrier adapted for topical administration. The pharmaceutical composition may include either aFGF or bFGF or a combination of the two. Fibroblast growth factor may be administered either with or without heparin, preferably with heparin when aFGF is included in the pharmaceutical composition. Topical administration of FGF, either aFGF or bFGF alone or in combination co-administered with PGFs will be in a solution, cream, ointment, gel, lotion, shampoo or aerosol formulation adapted for application to the skin. These topical pharmaceutical compositions containing compounds or agents demonstrating hair growth promoting activity will ordinarily include about 0.01 μg to about 100 μg/day/cm $^2$ preferably about 0.1 μg to about 10 μg/day/cm$^2$ of FGF and about 0.5 percent to about 5.0 percent of the compounds or agents demonstrating hair growth promoting activity in a pharmaceutically acceptable carrier. Combinations of FGF and compounds or agents demonstrating hair growth promoting activity may include any ratio of the components that will effectively stimulate hair growth or retard hair loss.

For topical application, various other pharmaceutical formulations or carriers are useful for the administration of the active compound of this invention. Such formulations include, but are not limited to the following: ointments such as hydrophilic petrolatum or polyethylene glycol ointment; pastes which may contain gums such as xanthan gum; solutions such as alcoholic, aqueous or buffered solutions; gels such as aluminum hydroxide or sodium alginate gels; albumins such as human or animal albumins; collagens such as human or animal collagens; celluloses such as alkyl celluloses, hydroxyalkyl celluloses and alkylhydroxyalkyl celluloses, for example methylcellulose, hydroxyethyl cellulose, carboxymethyl cellulose, hydroxypropyl methylcellulose, and hydroxypropyl cellulose; polyamers such as Pluronic® Polyols exemplified by Pluronic F-127; tetronics such as tetronic 1508; and alginates such as sodium alginate.

The present invention also relates to the in vitro pretreatment of hair plugs with FGF alone or in combination and with other PGFs or compounds or agents demonstrating hair growth promoting activity. The donor hair plugs are usually 6 mm full-thickness punch biopsies taken from the occipital region of the scalp of an individual, treated and implanted into pre-punched bald areas on either the vertex or frontal areas of the scalp and sutured into place. The hair follicles contained in the hair plugs or punch biopsies taken from the occipital region are not genetically pre-programed to fall out during the balding process and if adequately treated should grow following transplantation. The in vitro treatment of the hair plugs is carried out as described above for hair follicles. The treatment of the hair plugs with growth promoting agents will aid follicle growth and also stimulate general dermal vascularity and improve the survival of the transplants.

Preparation of hair plugs for transplantation will include multiple rinses with sterile water or physiological saline followed by a brief treatment with one or more FGF and or with protein growth factor or growth factors and any other hair growth promoter deemed necessary. If aFGF is administered the heparin should also be included. The plugs are immediately implanted and sutured in place. Follow up topical treatment with the above mentioned substances will further enhance the graft takes.

The following examples illustrate the present invention without, however, limiting the same thereto.

## EXAMPLE 1

Anagen stage human hair follicles were obtained from healthy human volunteers by mechanically removing individual follicles. The follicles were washed several times with Dulbecco's Modified Eagle Medium (DMEM) containing 10% heat inactivated fetal calf serum, 10,000 units penicillin G and 10,000 μg streptomycin sulfate fungizone. The washed follicles were placed in 24 well Nunc tissue culture plates, 3 follicles/well, and incubated

in a 5% $CO_2$ atmosphere at 37° C for 1 to 3 days. Each well contained 1.0 DMEM. Designated sample wells, in triplicate, were treated with 1.0, 0.1, 0.01 and 0.0 ug aFGF/ml per day from a lot containing 400 ug aFGF per ml of phosphate buffered saline. Heparin and human serum albumin (HSA) were added to each well to maintain a 3:1 heparin to aFGF ratio and a 10:1 HSA to aFGF ratio. $^3$H-thymidine, 5 uCi, 0.14 ug/well, and cold thymidine, 14.2 ug/well, were present during the final 24 hour period of incubation. Hair follicles were washed three times with ice cold 5% trichloroacetic acid (TCA) to remove unincorporated $^3$H-thymidine and to precipitate DNA. The protein precipitate was digested overnight at 45° C in IN NaOH and the amount of radiolabel incorporated into DNA determined by standard procedures.

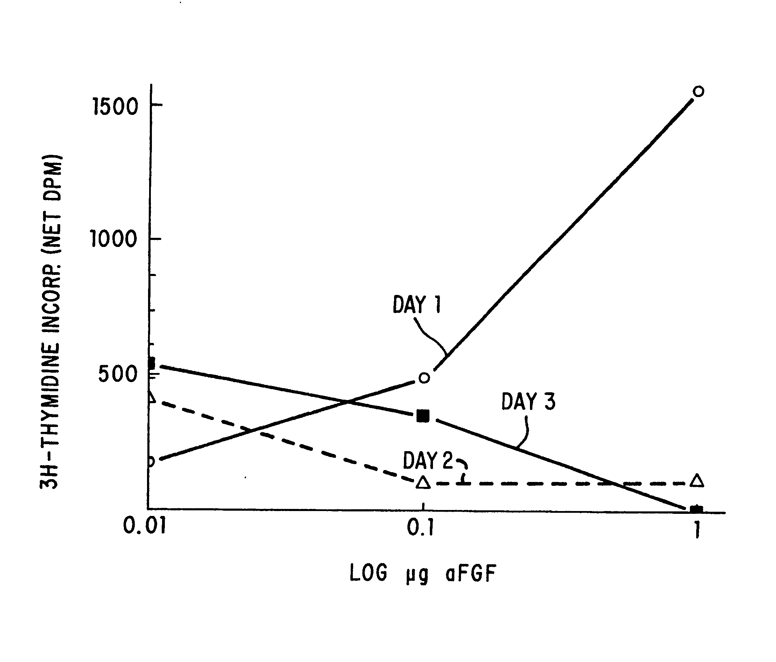

The level of $^3$H-thymidine incorporation into hair follicle DNA is shown in Figure 1. A dose response relationship was evident for those hair follicles incubated for 1 day with aFGF. This response was not evident in the Day 2 and 3 samples because of deterioration of the hair follicles in these cultures.

## EXAMPLE 2

Solution

A buffered solution is prepared by adding of 0.005% (by weight) of the protein growth factor or factors to phosphate buffer, pH 7.4. If aFGF is included then an amount of heparin equal to three times that of the growth factor is also added. If a hair growth promoting agent such as minoxidil is included then 0.1% (by weight) is added.

## EXAMPLE 3

Topical Cleaner

A topical cleaner is prepared by adding of 0.005% (by weight) of the protein growth factor or factors to the following formulation. If aFGF is included then an amount of heparin equal to three times that of the growth factor is also added. If a hair growth promoting agent such a minoxidil is included then 0.1% (by weight) is added.

```
Water                      80.439% by weight
Chamomile                   0.01
Allantoin                   0.001
Triethanolomine             0.03
METHOCEL® 40-100 (Dow)      1.50
Glycerine                   3.00
Sodium lauryl sulfate      15.00
Vitamine A oil              0.01
Vitamine E oil              0.001
```

## EXAMPLE 4

Cleansing Cream

A cleansing cream is prepared by adding of 0.005% (by weight) of the protein growth factor or factors to the following formulation. If aFGF is included then an amount of heparin equal to three times that of the growth factor is also added. If a hair growth promoting agent such as minoxidil is included then 0.1% (by weight) is added.

| | |
|---|---|
| Synthetic beeswax | 14.0% by weight |
| PPG2 Myristyl propionate | 5.0 |
| Lanolin alcohol | 0.5 |
| Mineral oil | 36.0 |
| Propyl paraben | 0.15 |
| Sodium borate | 1.0 |
| Water | 44.25 |

EXAMPLE 5

Skin Gel

A skin gel is prepared by adding of 0.005% (by weight) of the protein growth factor or factors to the following formulation. If aFGF is included then an amount of heparin equal to three times that of the growth factor is also added. If a hair growth promoting agent such as minoxidil is included then 0.1% (by weight) is added.

| | |
|---|---|
| PPG2 Myristyl ether propionate | 45.00% by weight |
| PPG10 Cetyl ether | 5.00 |
| C18-C36 triglyceride | 4.00 |
| Myristyl myristate | 3.00 |
| Glyceryl tribebenate | 2.00 |
| Cyclomethicone | 36.00 |
| Polyethylene | 5.00 |

EXAMPLE 6

Skin Lotion

A skin lotion is prepared by adding of 0.005% (by weight) of the protein growth factor or factors to the following formulation. If aFGF is included then an amount of heparin equal to three times that of the growth factor is also added. If a hair growth promoting agent such as minoxidil is included then 0.1% (by weight) is added.

| | |
|---|---|
| DEA Oleth 3 phosphate | 1.0% by weight |
| Emulsifying wax | 2.0 |
| C18-C36 wax fatty acids | 1.0 |
| PPG2 Myristyl propionate | 5.0 |
| Glycerine | 3.0 |
| Triethanolamine | 0.5 |
| Water | 87.5 |

### EXAMPLE 7

Shampoo Gel

A shampoo gel is prepared by adding of 0.05% (by weight) of the protein growth factor or factors to the following formulation. If aFGF is included then an amount of heparin equal to three times that of the growth factor is also added. If a hair growth promoting agent such as minoxidil is included then 0.1% (by weight) is added.

```
Isopropanolamine lauryl sulfate 83.5% by weight
Cocoamide DEA                    8.0
C18-C36 Was acid glyeryl ester   4.5
PPG5 Ceteth 10 phosphate         4.0
```

### EXAMPLE 8

Cream Shampoo

A cream shampoo is prepared by adding of 0.005% (by weight) of the protein growth factor or factors to the following formulation. If aFGF is included then an amount of heparin equal to three times that of the growth factor is also added. If a hair growth promoting agent such as minoxidil is included then 0.1% (by weight) is added.

```
Sodium Lauryl sulfate      65.0% by weight
Glyceryl tribebenate        2.0
Hydrolysed collagen         1.0
Lauric diethanolamide       5.0
Water                      27.0
```

## Claims

1. A method of improving the bodily appearance of a person which comprises topically applying to the scalp of said person one or more fibroblast growth factors in an amount effective to treat or prevent alopecia.

2. The method according to Claim 1 wherein the fibroblast growth factor is selected from the group consisting of acidic fibroblast growth factor, basic fibroblast growth factor, int-2, hst/KS3, FGF-5, FGF-6 or KGF.

3. The method according to Claim 2 wherein the fibroblast growth factor is acidic fibroblast growth factor.

4. The method according to Claim 2 or Claim 3 wherein the fibroblast growth factor is co-administered with one or more protein growth factors selected from the group consisting of epidermal growth factor, platelet-derived growth factor, transforming growth factor-alpha, transforming growth factor-beta, insulin-like growth factor-I and insulin-like growth factor-II.

5. The method according to Claim 2 or Claim 3 wherein the fibroblast growth factor is co-administered with one or more hair growth promoting compounds selected from the group consisting of minoxidil, minoxidil analogs, minoxidil derivatives, anti-androgens and 5α-reductase inhibitors.

6. The method according to Claim 1 wherein the fibroblast growth factor is topically applied in a pharmaceutical composition containing a pharmaceutically acceptable carrier.

7. The method according to Claim 4 wherein the fibroblast growth factor co-administered with protein growth factor is topically applied in a pharmaceutical composition containing a pharmaceutically acceptable carrier.

8. The method according to Claim 5 wherein the fibroblast growth factor co-administered with hair growth promoting compounds is topically applied in a pharmaceutical composition containing a pharmaceutically acceptable carrier.

9. The method according to any one of Claims 6 to 8 wherein the pharmaceutical composition is a solution, cleaner, cream, gel, lotion, ointment, shampoo or aerosol.

10. A method of improving the bodily appearance of a person which comprises the sequence of steps of:
   a. the removal of hair plugs from the occipital region of the scalp;
   b. treating the hair plugs or transplant recipient sites with one or more fibroblast growth factors in an effective stimulatory amount to treat or prevent alopecia;
   c. implantation and attachment of the treated hair plugs into the vertex or frontal areas of the scalp.

11. The method according to Claim 10, step b wherein the fibroblast growth factor is selected from the group consisting of acidic fibroblast growth factor, basic fibroblast growth factor, int-2, hst/KS3, FGF-5, FGF-6 or KGF.

12. The method according to Claim 10, step b or Claim 11 wherein the fibroblast growth factor is co-administered with one or more protein growth factors selected from the group consisting of epidermal growth factor, platelet-derived growth factor, transforming growth factor-alpha, transforming growth factor-beta, insulin-like growth factor-I and insulin-like growth factor-II.

13. The method according to Claim 10, step b or Claim 11 wherein the fibroblast growth factor is co-administered with one or more hair growth promoting compounds selected from the group consisting of minoxidil, minoxidil analogs, minoxidil derivatives, anti-androgens and 5α-reductase inhibitors.

14. The use of one or more fibroblast growth factors for the manufacture of a medicament suitable for treating or preventing alopecia.

15. The use as claimed in Claim 14 wherein the medicament is adapted for co-administration of the fibroblast growth factor with one or more protein growth factors selected from the group consisting of epidermal growth factor, platelet-derived growth factor, transforming growth factor-alpha, transforming growth factor-beta, insulin-like growth factor-I and insulin-like growth factor-II.

16. The use as claimed in Claim 14 wherein the medicament is adapted for co-administration of the fibroblast growth factor with one or more hair growth promoting compounds selected from the group consisting of minoxidil, minoxidil analogs, minoxidil derivatives, anti-androgens and 5α-reductase inhibitors.